(19) Europäisches Patentamt
European Patent Office
Office européen des brevets

(11) Veröffentlichungsnummer : **0 091 464**
**B1**

(12) # EUROPÄISCHE PATENTSCHRIFT

(45) Veröffentlichungstag der Patentschrift : **16.04.86**

(51) Int. Cl.⁴ : **C 07 D213/84**, C 07 D215/48, C 07 D217/26

(21) Anmeldenummer : **82903166.5**

(22) Anmeldetag : **14.10.82**

(86) Internationale Anmeldenummer : **PCT/DE 82/00206**

(87) Internationale Veröffentlichungsnummer : **WO/8301446 (28.04.83 Gazette 83/10)**

(54) **VERFAHREN ZUR HERSTELLUNG VON CYANOHETEROCYCLEN.**

(30) Priorität : **16.10.81 DE 3141640**
**19.08.82 DE 3231072**

(43) Veröffentlichungstag der Anmeldung : **19.10.83 Patentblatt 83/42**

(45) Bekanntmachung des Hinweises auf die Patenterteilung : **16.04.86 Patentblatt 86/16**

(84) Benannte Vertragsstaaten : **AT BE CH DE FR GB LI LU NL SE**

(56) Entgegenhaltungen :
**US-A- 2 991 285**
**Chemical Abstracts, Band 92, Nr. 23, 9. Juni 1980, (Columbus, Ohio, US), M. ODA "One-pot transformation of ketones to alpha,beta-unsaturated nitriles via trimethylsiloxy nitriles", Seite 693, Zusammenfassung 197313h**
**Die Akte enthält technische Angaben, die nach dem Eingang der Anmeldung eingereicht wurden und die nicht in dieser Patentschrift enthalten sind.**

(73) Patentinhaber : **SCHERING AKTIENGESELLSCHAFT Berlin und Bergkamen**
**Müllerstrasse 170/178 Postfach 65 03 11**
**D-1000 Berlin 65 (DE)**

(72) Erfinder : **VORBRÜGGEN, Helmut**
**Wilkestrasse 7**
**D-1000 Berlin 27 (DE)**

**EP 0 091 464 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Heterocyclische N-Oxide wie Pyridin-, Chinolin- Iso-chinolin- oder Pyrimidin-N-oxid lassen sich nach O-Alkylierung oder Acylierung und anschließender Behandlung mit wäßriger Cyanidlösung in die entsprechenden 2- oder 4-substituierten Cyanoverbindungen umwandeln (US-Patent 2,991,285). Bei den weniger reaktiven Pyridin-N-oxiden entstehen oft nur in schlechten Ausbeuten Gemische von 2- und 4-Cyanoverbindungen (E. Ochiai, Aromatic Amine Oxides, Elsevier Publishing Co., Amsterdam 1968, A.R. Katritzky, J.M. Lagowski, Chemistry of the Heterocyclic N-Oxides, Academic Press London-New York 1971). M. Oda et al. beschreiben in Chem. Lett. 1979. 1427 die Umsetzung von Ketonen mit Trimethylsilylcyanid in Gegenwart von $ZnI_2$ und anschließendes Erhitzen nach Zugabe von $POCl_3$/Pyridin zu $\alpha,\beta$-ungesättigten Nitrilen.

Es wurde nun gefunden, daß sich heterocyclische N-Oxide in nur einer Reaktionsstufe und in hohen Ausbeuten selektiv in die entsprechenden 2-Cyanoheterocyclen umwandeln lassen, wenn man die heterocyclischen N-Oxide in Gegenwart eines tertiären Amins entweder mit Trimethylsilylcyanid ohne Lösungsmittel oder in einem inerten Lösungsmittel erhitzt bzw. ökonomischer das heterocyclische N-Oxid mit einem Alkalicyanid und Trimethylsilylchlorid in einem polaren Lösungsmittel erhitzt, d. h. das Trimethylsilylcyanid *in situ* erzeugt.

Die Erfindung betrifft somit Verfahren zur Herstellung von Cyanoheterocyclen der allgemeinen Formel I

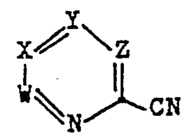

(I)

worin W, X, Y und Z ein Stickstoffatom, den Rest CH oder den Rest $CR_1$ mit $R_1$ in der Bedeutung eines Wasserstoffatomes, eines geradkettigen oder verzweigten Alkylrestes mit 6-10 C-Atomen, der gegebenenfalls substituiert sein kann, eines Aralkyls mit 7-10 C-Atomen, Aryls, Heteroaryls, Carboxyls, Hydroxyrestes, Aminorestes, Mono- oder Dialkylaminorestes, Mono- oder Diarylaminorestes, Alkylsulfonyls, Arylsulfonyls, Trifluormethyls, eines Halogensubstituenten eines Cycloalkyls, einer Cyanogruppe, einer Gruppe $—OR_2$ mit $R_2$ als geradkettigen oder verzweigten Kohlenwasserstoffrest mit 1-10 C-Atomen, als Cycloalkylrest mit 3-10 C-Atomen, Aralkyl mit 7-10 C-Atomen, Aryl oder Heteroaryl, einer Gruppe $—COOR_3$ mit $R_3$ als geradkettigen oder verzweigten Kohlenwasserstoffrest mit 1-10 C-Atomen, als Cycloalkylrest mit 3-10 C-Atomen, Aralkyl mit 7-10 C-Atomen, Phenyl, Naphthyl oder Heteroaryl oder einer Gruppe $—CONR_4R_5$ mit $R_4$ und $R_5$ als Wasserstoff, geradkettigen oder verzweigten Kohlenwasserstoffrest mit 1-10 C-Atomen, Cycloalkylrest mit 3-10 C-Atomen, Aralkyl mit 7-10 C-Atomen, Aryl oder Heteroaryl oder $R_4$ als Wasserstoff und $R_5$ als $—COR_6$ oder $—SO_2R_6$ mit $R_6$ in der Bedeutung von $R_2$ darstellen oder W—X, X—Y und Y—Z Teil eines gegebenenfalls mit $R_1$ in der angegebenen Bedeutung substituierten aliphatischen, aromatischen oder heterocyclischen Ringes, der ein oder mehrere Stickstoff-, Sauerstoff- oder Schwefelatome enthalten kann, sind, dadurch gekennzeichnet, daß man ein N-Oxid der allgemeinen Formel II

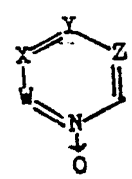

(II)

mit den für W, X, Y, Z und $R_1$ angegebenen Bedeutungen mit Trimethylsilylcyanide umsetzt.

Die N-Oxide der allgemeinen Formel II werden in Gegenwart eines tert. Amins wie Triäthylamin entweder ohne Lösungsmittel oder in einem inerten Lösungsmittel wie Acetonitril, Pyridin, Dimethylformamid, N-Methylpyrrolidon, Tetrahydrofuran (THF) usw., bevorzugt in Acetonitril, mit ca. 2-3 Äquivalenten Trimethylsilylcyanid bzw. mit einem Alkalicyanid, wie Natrium- oder Kaliumcyanid, mit Trimethylchlorsilan in einem inerten polaren Lösungsmittel wie Dimethylformamid oder Methylpyrrolidon, z. B. auch in Gegenwart eines Phasentransferkatalysators wie z. B. Adogen[R] 464 bei 50-250 °C, bevorzugt bei 70-180 °C, umgesetzt, wobei sich nach Literaturangaben (S. Hünig, Synthesis 1979, 522) Trimethylsilylcyanid *in situ* bildet, so daß sich die Darstellung des teuren und relativ instabilen Reagenzes erübrigt.

Man kann die Reaktion des N-Oxids mit überschüssigem Trimethylsilylcyanid und Triäthylamin in absolutem Tetrahydrofuran (THF) auch schon bei 0-10 °C durchführen, wenn man katalytische Mengen eines in THF-löslichem Fluoridsalzes wie Tetra-n-butylammoniumfluorid zusetzt. Nur dürfen in einem solchen Falle keine weiteren silylierten reaktiven Gruppen im N-Oxid vorliegen wie z. B. die Trimethylsilyloxygruppe im silylierten 3-Hydroxypyridin-N-Oxid.

Als Alkylgruppen $R_1$-$R_6$ sind gerade oder verzweigte Alkylgruppen mit 1-10 C-Atomen gemeint, wie

beispielsweise Methyl, Äthyl, Propyl, Isopropyl, Butyl, Isobutyl, tert.-Butyl, Pentyl, Neopentyl, Heptyl, Hexyl, Decyl.

Die Alkylgruppen $R_1$-$R_6$ können gegebenenfalls 1- bis mehrfach substituiert sein durch Halogenatome, Alkoxygruppen, gegebenenfalls substituierte Arylgruppen, Dialkylamino und Trialkylammonium.

Bevorzugt sind solche Alkylgruppen, die einfach substituiert sind. Als Substituenten seien beispielsweise genannt Fluor-, Chlor- oder Bromatome, Phenyl, Dimethylamino, Diäthylamino, Methoxy, Äthoxy.

Als bevorzugte Alkylgruppen $R_1$-$R_6$ sind solche mit 1-4 C-Atomen, wie z. B. Methyl, Äthyl, Propyl, Dimethylaminopropyl, Isobutyl, Butyl zu nennen. Als Arylgruppen $R_1$-$R_6$ kommen sowohl substituierte wie auch unsubstituierte Arylgruppen in Betracht, wie beispielsweise Phenyl, 1-Naphthyl und 2-Naphthyl, die jeweils substituiert sein können durch 1-3 Halogenatome, eine Phenylgruppe, 1-3 Alkylgruppen mit jeweils 1-4 C-Atomen, eine Chlormethyl-, Fluormethyl-, Trifluormethyl-, Carboxyl-, Hydroxy- oder Alkoxygruppe mit 1-4 C-Atomen. Bevorzugt sind die Substituenten in 3- und 4-Stellung am Phenylring, zum Beispiel durch Fluor, Chlor, Alkoxy oder Trifluormethyl oder in 4-Stellung durch Hydroxy. Die Aralkylgruppe $R_1$-$R_6$ kann 7-10, vorzugsweise 7 und 8 Kohlenstoffatome, enthalten. Als Ar-Reste kommen unsubstituiertes Phenyl und substituiertes Phenyl in Betracht. Die Substituenten entsprechen den für $R_1$ als Arylgruppe. Als -alkyl-reste kommen geradkettige und verzweigte Alkylengreste mit 1-4 C-Atomen in Betracht. Als heterocyclische Gruppen $R_1$-$R_6$ kommen 5- und 6-gliedrige Heterocyclen infrage, die wenigstens 1-Heteroatom vorzugsweise Stickstoff, Sauerstoff oder Schwefel enthalten. Beispielsweise seien genannt : 2-Furyl, 3-Furyl, 2-Thienyl, 3-Thienyl, 2-Pyridyl, 3-Pyridyl, 4-Pyridyl u. a.

Als Alkylgruppen der Reste Monoalkylamino, Dialkylamino oder Alkylsulfonyl kommen gerad- oder verzweigtkettige Alkylreste mit 1-6, vorzugsweise mit 1-4 C-Atomen, infrage. Beispielsweise genannt seien Methyl-, Äthyl-, Propyl-, Butyl-, Isobutyl-, tert.-Butyl-, Pentyl-, Hexyl-.

Die Cycloalkylgruppe $R_1$-$R_6$ kann im Ring 4-10, vorzugsweise 5 und 6 Kohlenstoffatome enthalten. Die Ringe können durch Alkylgruppen mit 1-4 Kohlenstoffatomen substituiert sein. Beispeilsweise seien genannt Cyclopentyl-, Cyclohexyl-, Methyl-cyclohexyl und Adamantyl.

Als Arylgruppen der Reste Monoarylamino, Diarylamino oder Arylsulfonyl kommen beispielsweise in Betracht : Phenyl, 1-Naphthyl und 2-Naphthyl.

Als Halogene sind Fluor, Chlor und Brom gemeint.

Das Verfahren hat weiterhin den Vorteil, daß reaktive Gruppen wie Hydroxyl, Carboxyl und Aminogruppen während der Reaktion silyliert und dadurch geschützt werden. Diese Silylierung erleichtert auch die Aufarbeitung, da sich die meisten persilylierten Endprodukte nach Zusatz von Eiswasser durch Extraktion mit Hexan oder Toluol leicht von polaren Lösungsmitteln wie N-Methylpyrrolidon abtrennen lassen.

Als Ausgangsverbindungen II kommen folgende N-, O- oder S-haltige heterocyclische N-Oxyde, bestehend aus 1 Ring oder einem annelierten Ringsystem, in Betracht : Pyridin-N-oxyde, Pyridazin-N-oxyde, Pyrimidin-N-oxyde, Pyrazin-N-oxyde, Chinolin-N-oxyde, Isochinolin-N-oxyde, Acridin-N-oxyde, β- oder γ-Carbolin-N-oxyde, Purin-N-oxyde usw.

Heterocyclische N-Oxyde, die die genannten reaktiven Gruppen enthalten, lassen sich nach den eingangs erwähnten herkömmlichen Methoden nur schlecht in die entsprechenden Cyanderivate umwandeln, da nach den bekannten Methoden zuerst die N-Oxydgruppierung acyliert oder alkyliert werden muß, wobei gleichzeitig die reaktive Gruppe acyliert oder alkyliert wird, während nach dem erfindungsgemäßen Verfahren die reaktiven Gruppen durch die sehr leicht abspaltbare Silylgruppe geschützt werden.

Nach dem erfindungsgemäßen Verfahren läßt sich z. B. aus 3-Hydroxy-pyridin-N-oxyd in ca. 80 % Ausbeute das noch nicht beschriebene 5-Hydroxy-2-cyanopyridin herstellen, das ein wertvolles Ausgangsmaterial z. B. für die Synthese von Analoga der Fusarinsäure darstellt (DOS 29 11 492 und DAS 27 43 944).

## Beispiel 1

2-Cyanopyridin

Zu einer Lösung von 2,4 g frisch destilliertem Pyridin-N-oxyd und 5,0 ml Triäthylamin in 20 ml siedendem abs. Acetonitril wurde unter Rühren während 1 h 7,8 ml Trimethylsilylcyanid zugetropft. Nach weiteren 8 h Kochen und Stehen der Reaktionsmischung für 1 Woche bei 24°, wurden 10 ml Methanol zugegeben und nach 30 min eingeengt. Den Rückstand löste man in 50 ml Methylenchlorid und extrahierte mit ca. 50 ml eiskalter ges. $NaHCO_3$-Lösung. Nach Trocknen und Filtration der Methylenchloridphase wurde eingeengt. Der Rückstand wurde in wenig Äther aufgenommen und über eine Säule von 100 g neutralem Aluminiumoxyd (AII) filtriert. Die Eluate wurden eingeengt und der Rückstand am Kugelrohr destilliert, wobei 2,08 g (80 %) leicht gelbliches Destillat erhalten wurde, das nach der geschromatischen Analyse aus ca. 95 % 2-Cyano-pyridin und ca. 0,5 % 4-Cyanopyridin besteht.

## Beispiel 2

2-Cyano-6-methyl-pyridin

2,75 g 2-Methylpyridin-N-oxyd, 6,9 ml Trimethylsilylcyanid und 3,5 ml Triäthylamin wurden 30 h bei 100° Badtemperatur unter Ausschluß von Feuchtigkeit gekocht, 100 ml $H_2O$ zugefügt und 3 x mit 100 ml Methylenchlorid extrahiert. Die organische Phase wurde getrocknet und abgedampft und der Rückstand in Hexan-Essigester (9 : 1) über eine Säule von 150 g neutralem Aluminiumoxyd (AII) filtriert, wobei die ersten 600 ml Eluat nach Abdampfen und Umkristallisation aus Hexan 1,3 g (39 %) 2-Cyano-6-methylpyridin, Fp. 71,5-72° ergaben.

Beispiel 3

2-Cyano-4-methyl-pyridin

2,75 g 4-Methylpyridin-N-oxyd und 6,7 ml Triäthylamin wurden mit 12,52 ml Trimethylsilylcyanid in 10 ml abs. Acetonitril unter Feuchtigkeitsausschluß 20 h am Rückfluß gekocht. Nach Zugabe von 30 ml Methanol wurde abgedampft und der Rückstand in 100 ml $H_2O$ mit 3 × 100 ml Methylenchlorid extrahiert. Nach Trocknen der Extrakte und Abdampfen wurden 4,01 g Rohprodukt erhalten, das in Hexan-Essigester (9 : 1) über eine Säule von 150 g neutralem Aluminiumoxyd (AII) filtriert wurde. Die ersten 750 ml Eluat ergaben 2,66 g (89,3 %) leicht gelbliche Kristalle von 2-Cyano-4-methylpyridin, Fp. 89-91°.

Beispiel 4

2-Cyano-5-hydroxy-pyridin

a) 2,78 g 3-Hydroxy-pyridin-N-oxyd, 11 ml Trimethylsilylcyanid und 8,3 ml Triäthylamin in 20 ml abs. Acetonitril kochte man 8 h unter Feuchtigkeitsausschluß am Rückfluß. Nach Zugabe von 5 ml Hexamethyldisilazan wurde 30 Min. gekocht und am Rotationsverdampfer eingeengt. Den Rückstand destillierte man bei 120-130°/2 mbar im Kugelrohr und versetzte das Destillat mit Methanol, wobei unter Erwärmen rohes 2-Cyano-5-hydroxy-pyridin kristallisierte. Nach Umkristallisation aus Essigester unter Zusatz von Tierkohle wurden 2,19 g (73 %) reines 2-Cyano-5-hydroxy-pyridin, Fp. 210° (Zers.) erhalten.

b) Zu einer Suspension von 3,33 g 3-Hydroxypyridin-N-oxyd, 2,94 g Natriumcyanid und 20,8 ml Triäthylamin in 40 ml abs. DMF wurde unter Rühren und Wasserkühlung innerhalb von 1 h 15, 1 ml Trimethylchlorsilan zugetropft und die Reaktionsmischung auf 110-120° Ölbad-Temperatur erwärmt. Nach Abkühlen auf 24°, Filtration der Salze und Nachwaschen mit 50 ml DMF wurde das Filtrat eingeengt. Nach Zugabe von Methanol erwärmte sich der Rückstand, der nach erneutem Einengen aus 100 ml Essigester unter Zusatz von Kohle umkristallisiert wurde. Man erhielt 3,21 g (90,1 %) reines 2-Cyano-5-hydroxy-pyridin.

Beispiel 5

2-Cyano-5-carboxy-pyridin

4,17 g Nicotinsäure-N-oxyd, 4,41 g Natriumcyanid und 20,8 ml Triäthylamin wurden in 100 ml abs. DMF suspendiert und unter Kühlen und Rühren 15,1 ml Trimethylchlorsilan während einer Stunde zugetropft, wobei sich die Suspension orangerot färbte. Nach 10 h Erhitzen auf 100-110° Badtemperatur und Abkühlen auf 23° filtrierte man die unlöslichen Salze ab, wusch mit 50 ml abs. DMF und engte das Filtrat bei 60-65°/12 mm ein. Der braune kristalline Rückstand wurde in 120 ml 1 N HCl gelöst und mit 6 × 100 ml Methylenchlorid extrahiert. Nach Trocknen und Abdampfen der Methylenchloridphase wurde der Rückstand in 100 ml $H_2O$ heiß gelöst, mit Kohle behandelt, filtriert und eingeengt, wobei 3,3 g (76 %) 2-Cyano-5-carboxypyridin vom Fp. 198-201° erhalten wurden.

Beispiel 6

2-Cyanochinolin

4,9 g Chinolin-N-oxyd-monohydrat, 2,94 g Natriumcyanid und 24,93 ml Triäthylamin wurden in 60 ml abs. DMF gerührt und 19 ml Trimethylchlorsilan während 45 min zugetropft, wobei sich die Lösung anfangs auf 40° erwärmte. Durch Kühlung wurde die Temperatur dann auf 24° gehalten. Nach 3 h Erhitzen auf 100-110° Ölbadtemperatur wurde auf 25° gekühlt, die Salze abfiltriert und mit 60 ml DMF nachgewaschen. Nach Zugabe von 20 ml Methanol dampfte man die organische Phase ab und filtrierte den gelbbraunen kristallinen Rückstand (7 g) über 140 g neutrales Aluminiumoxyd (AII). Elution mit 250 ml Hexan-Methylenchlorid (1 : 1) lieferte 4,2 g (90,1 %) farbloses kristallines 2-Cyanochinolin, das aus ca. 150 ml Hexan umkristallisiert wurde und in mehreren Portionen reines 2-Cyanochinolin, 94,7° lieferte.

Beispiel 7

1-Cyanoisochinolin

2,9 g Isochinolin-N-oxyd wurde in 10 ml abs. N-Methylpyrrolidon gelöst und 7,5 ml Trimethylsilylcyanid zugegeben, wobei sich die Reaktionsmischung auf 50° erwärmte. Nach 17 h bei 24° wurde auf 300 ml $H_2O$ gegossen, die ausgefallenen leicht grauen Kristalle abgesaugt und im Vakuum getrocknet, wobei 2,58 g (82,7 %) rohes 1-Cyanoisochinolin erhalten wurden. Umkristallisation aus ca. 200 ml Hexan ergab in mehreren Portionen 2,46 g (78,9 %) reine Substanz, Fp. 90°.

Beispiel 8

2-Cyano-3-methyl-pyridin und 2-Cyano-5-methylpyridin

Zu einer Suspension von 3,27 g 3-Methylpyridin-N-oxyd, 4,41 g Natriumcyanid und 16,6 ml Triäthylamin in 100 ml abs. DMF tropfteman unter Rühren während 1 h 11,42 Trimethylchlorsilan und erhitzte anschließend 52 h auf 100-110° Ölbadtemperatur. Nach Abkühlen wurde die schwarze Lösung filtriert, mit wenig Äther nachgewaschen und das Filtrat bei 12 mm abgedampft. Der teilweise kristalline Rückstand (8,2 g) wurde in Toluol an 240 g neutralem $Al_2O_3$ (AII) chromatographiert. Die ersten 750 ml Eluat ergaben 3,05 g (86 %) kristallines Gemisch, aus dem durch einfache fraktionierte Kristallisation aus Hexan ca. 1,4 g (40 %) reines 2-Cyano-5-methylpyridin vom Fp. 73-75 (DC-System Toluol-Essigester = 1 : 1, Rf = 0,47) und ca. 1,4 g (40 %) reines 2-Cyano-3-methylpyridin vom Fp. 84-86° (Rf = 0,43) isoliert wurden.

Beispiel 9

2-Cyano-5-ethoxycarbonyl-pyridin

Analog Beispiel 1 erhielt man aus 5 g Nikotinsäurethylester-N-oxid 4,12 g (78 % der Theorie) 2-Cyano-5-ethoxycarbonyl-pyridin vom Fp. 56°.

Beispiel 10

2-Cyano-5-phenoxy-pyridin

Zu einer Suspension von 5,6 g 5-Phenoxy-pyridin-N-oxid (Herstellung beschrieben in US 4.212.980), 2,94 g Natriumcyanid und 20,8 ml Triethylamin in 40 ml DMF wurden unter Rühren und Wasserkühlung innerhalb von 1 h 15,1 ml Trimethylchlorsilan zugetropft und die Reaktionsmischung auf 110-120° Ölbad-Temperatur 8 h lang erwärmt. Nach Abkühlen auf 24°, Filtration der Salze und Nachwaschen mit 50 ml DMF wurde das Filtrat eingeengt und in Hexan-Essigester (9 : 1) an Aluminiumoxid (AII) chromatographiert. Ausbeute 4,27 g (73 % der Theorie) vom Fp. 84-85°.

Beispiel 11

2-Cyano-5-hydroxymethyl-pyridin

Analog zu Beispiel 10 erhielt man aus 3,75 g 3-Hydroxymethylpyridin-N-oxid [nach S. Furuoka in Yakugaku Zasshi *78,* 957 (1968)] 2,69 g (67 % der Theorie) vom Fp. 78-79°.

Beispiel 12

2-Cyano-5-carboxamidopyridin

Zu einer Suspension von 4,14 g Nicotinsäureamid-N-oxid, 4,41 g Natriumcyanid und 25,1 ml Triethylamin in 150 ml abs. DMF wurden 19,02 ml Trimethylchlorsilan unter Rühren innerhalb von 45 min. zugetropft und anschließend 12 h unter Wasserausschluß gekocht. Nach Abkühlen, Filtration und Nachwaschen mit 40 ml DMF wurde das Filtrat im Vakuum eingeengt und der braune, teils kristalline Rückstand mit 40 ml $H_2O$ auf 40° erwärmt, gekühlt und das ausgefallene Endprodukt abfiltriert und mit 20 ml $H_2O$ gewaschen. Nach Umkristallisation aus 120 ml Methanol-$H_2O$ (1 : 1) unter Zusatz von Tierkohle und Einengen wurden in mehreren Portionen 3,1 g (70,3 %) reines 2-Cyano-5-carboxamido-pyridin vom Fp. 243-250° (Zers.) isoliert.

Beispiel 13

2,3-Dicyano-pyridin und 2,5-Dicyanopyridin

Zu einer Suspension von 5,4 g 3-Cyanopyridin-N-oxid, 6,61 g Natriumcyanid und 25 ml Triethylamin in 100 ml abs. DMF wurden unter Rühren 17,13 ml Trimethylchlorsilan innerhalb von 10 min. unter Rühren zugetropft und anschließend 8 h bei 100-105° Ölbadtemperatur am Rückfluß gekocht. Danach konnten im Dünnschichtchromatogramm (System Toluol-Essigester = 1:1) nur noch Spuren Ausgangsmaterial nachgewiesen werden. Nach Abkühlen wurden die Salze abfiltriert, mit wenig DMF nachgewaschen und das Filtrat bei 12 mm Vakuum eingeengt. Den Rückstand schüttelte man mit 150 ml $CH_2Cl_2$ und 50 ml eiskalter 2 N NaOH-Lösung und extrahierte die wäßrige Phase noch zweimal mit jeweils 50 ml $CH_2Cl_2$. Die vereinigten $CH_2Cl_2$-Extrakte wurden nach Trocknung eingeengt. Das ölige Rohprodukt (7,7 g) chromatographierte man in Toluol an einer Säule von 220 g Silicagel, wobei nach Elution mit Toluol-Essigester (9 : 1) zuerst 2,5-Dicyanopyridin (Rf = 0,56) und nach weiterer Elution 2,3-Dicyanopyridin (Rf = 0,41) erhalten wurde. Kristallisation der reinen und einfache fraktionierte Kristallisation der Mischfraktionen aus Äther-Hexan ergaben ca. 1,6 g (27,5 %) reines 2,5-Dicyanopyridin vom Fp. 102,5° und ca. 3,1 g (53,4 %) 2,3-Dicyanopyridin vom Fp. 79,5°.

Beispiel 14

2,3-Dicyanopyridin und 2,5-Dicyanopyridin

Zu einer eisgekühlten Suspension von 1,2 g (10m mol) 3-Cyanopyridin-N-Oxid, 5,58 ml (40m mol) Triäthylamin und 1 ml einer 1 molaren Lösung von wasserfreiem Tetra-n-butylammoniumfluorid in Tetrahydrofuran in 40 ml abs. Tetrahydrofuran wurden 3,76 ml (30m mol) Trimethylsilylcyanid in 5 ml Tetrahydrofuran bei + 50 °C während 30 Min. unter Rühren zugetropft und noch weitere 30 Min. bei + 5°C gerührt. Nach Abdampfen und Aufarbeiten mit Methylenchlorid-wässriger $NaHLO_3$-lösung wurde das Rohprodukt (2,2 g) in wenig Toluol-$CH_2Cl_2$ gelöst und an einer Säule von 75 g Silicagel mit Toluol bzw. Toluol-Essigester (95 : 5) chromatographiert, wobei 0,23 g (17,8 %) reines 2,5-Dicyanopyridin sowie 0,62 g (48 %) reines 2,3-Dicyanopyridin erhalten wurden.

**Patentanspruch**

Verfahren zur Herstellung von Cyanoheterocyclen der allgemeinen Formel I

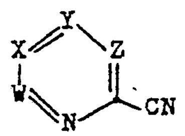

(I)

worin W, X, Y und Z ein Stickstoffatom, den Rest CH oder den Rest $CR_1$ mit $R_1$ in der Bedeutung eines Wasserstoffatomes, eines geradkettigen oder verzweigten Alkylrestes mit 1-10 C-Atomen, der gegebenenfalls substituiert sein kann, eines Aralkyls mit 7-10 C-Atomen, Aryls, Heteroaryls, Carboxyls, Hydroxyrestes, Aminorestes, Mono- oder Dialkylaminorestes, Mono- oder Diarylaminorestes, Alkylsulfonyls, Arylsulfonyls, Trifluormethyls, eines Halogensubstituenten, eines Cycloalkyls, einer Cyanogruppe, einer Gruppe $—OR_2$ mit $R_2$ als geradkettigen oder verzweigten Kohlenwasserstoffrest mit 1-10 C-Atomen, als Cycloalkylrest mit 3-10 C-Atomen, Aralkyl mit 7-10 C-Atomen, Aryl oder Heteroaryl, einer Gruppe $—COOR_3$ mit $R_3$ als geradkettigen oder verzweigten Kohlenwasserstoffrest mit 1-10 C-Atomen, als Cycloalkylrest mit 3-10 C-Atomen, Aralkyl mit 7-10 C-Atomen, Phenyl, Naphthyl oder Heteroaryl oder einer Gruppe $—CONR_4R_5$ mit $R_4$ und $R_5$ als Wasserstoff, geradkettigen oder verzweigten Kohlenwasserstoffrest mit 1-10 C-Atomen, Cycloalkylrest mit 3-10 C-Atomen, Aralkyl mit 7-10 C-Atomen, Aryl oder Heteroaryl oder $R_4$ als Wasserstoff und $R_5$ als $—COR_6$ oder $—SO_2R_6$ mit $R_6$ in der Bedeutung von $R_2$ darstellen oder W-X, X-Y und Y-Z Teil eines gegebenenfalls mit $R_1$ in der angegebenen Bedeutung substituierten aliphatischen, aromatischen oder heterocyclischen Ringes, der ein oder mehrere Stickstoff-, Sauerstoff- oder Schwefelatome enthalten kann, sind, dadurch gekennzeichnet, daß man ein N-Oxid der allgemeinen Formel II

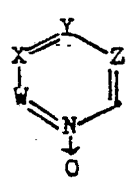

(II)

mit den für W, X, Y, Z und $R_1$ angegebenen Bedeutungen in Gegenwart eines tertiären Amins mit Trimethylsilylcyanid umsetzt.

**Claim**

Process for the preparation of cyano heterocyclic compounds of general formula I

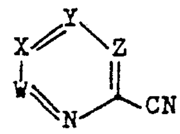

(I)

wherein W, X, Y and Z are nitrogen, CH or $CR_1$, in which $R_1$ is hydrogen, a straight or branched chain alkyl of 1 to 10 carbon atoms, that may be substituted, an aralkyl group of 7-10 carbon atoms, aryl, heteroaryl, carboxy, hydroxy, amino, mono- or dialkylamino, mono- or diarylamino, alkylsulphonyl, arylsulphonyl, trifluoromethyl, halogen, cycloalkyl, cyano, $—OR_2$ (where $R_2$ is straight or branched chain hydrocarbyl of 1 to 10 carbon atoms, cycloalkyl of 3-10 carbon atoms, aralkyl of 7-10 carbon atoms, aryl or heteroaryl), $—COOR_3$ (where $R_3$ is straight or branched chain hydrocarbyl of 1-10 carbon atoms, cycloalkyl of 3-10 carbon atoms, aralkyl of 7-10 carbon atoms, phenyl, naphthyl or heteroaryl) or $—CONR_4R_5$ where $R_4$ and $R_5$ are hydrogen, straight or branched chain hydrocarbyl of 1-10 carbon atoms, cycloalkyl of 3-10 carbon atoms, aralkyl of 7-10 carbon atoms, aryl or heteroaryl or $R_4$ is hydrogen and $R_5$ is $—COR_6$ or $—SO_2R_6$ (where $R_6$ has the same meaning as $R_2$), or W-X, X-Y and Y-Z are parts of an aliphatic, aromatic or heterocyclic ring, optionally substituted by $R_1$ (which has the meaning given above), and which can contain one or more nitrogen, oxygen or sulphur atoms, characterised in that an N-oxide of general formula II

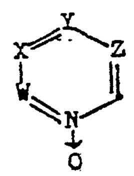

(II)

in which W, X, Y, Z and $R_1$ have the meanings given above, is treated with trimethylsilylcyanide, in the presence of a tertiary amine.

**Revendication**

Procédé de préparation de cyano-hétérocycles de formule générale I ci-dessous

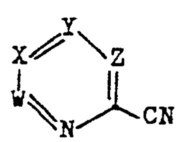

(I)

dans laquelle W, X, Y et Z désignent chacun un atome d'azote, le radical CH, un radical $CR_1$, $R_1$ étant un atome d'hydrogène, un alkyle à chaîne droite ou ramifiée en $C_1$ à $C_{10}$ pouvant avoir éventuellement des substituants, un aralkyle en $C_7$ à $C_{10}$, un aryle, un hétéroaryle, un carboxyle, un hydroxyle, un groupe amino, mono- ou di-alkylamino, mono- ou di-arylamino, alkylsulfonyle, arylsulfonyle, trifluorométhyle, un halogène, un cycloalkyle, un groupe cyano, un groupe $—OR_2$, $R_2$ étant un radical hydrocarboné à chaîne droite ou ramifiée en $C_1$ à $C_{10}$, un cycloalkyle en $C_3$ à $C_{10}$, un aralkyle en $C_7$ à $C_{10}$, un aryle ou un hétéroaryle, un groupe $—COOR_3$, $R_3$ étant un radical hydrocarboné à chaîne droite ou ramifiée en $C_1$ à $C_{10}$, un cycloalkyle en $C_3$ à $C_{10}$, un aralkyle en $C_7$ à $C_{10}$, un phényle, un naphtyle ou un hétéroaryle, ou encore un groupe $—CONR_4R_5$, $R_4$ et $R_5$ étant chacun l'hydrogène, un radical hydrocarboné à chaîne droite ou ramifiée en $C_1$ à $C_{10}$, un cycloalkyle en $C_3$ à $C_{10}$, un aralkyle en $C_7$ à $C_{10}$, un aryle ou un hétéroaryle, ou bien $R_4$ est l'hydrogène et $R_5$ un radical $—COR_6$ ou $—SO_2R_6$, $R_6$ ayant la même signification que $R_2$ ci-dessus, ou bien W-X, X-Y et Y-Z forment une partie d'un cycle aliphatique ou aromatique ou d'un hétérocycle, éventuellement substitué par un radical $R_1$ tel que défini ci-dessus et pouvant comporter un ou plusieurs atomes d'azote, d'oxygène ou de soufre), procédé caractérisé en ce que l'on fait réagir un N-oxyde de formule générale II

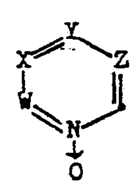

(II)

avec le cyanure de triméthylsilyle en présence d'une amine tertiaire.